# EUROPÄISCHE PATENTANMELDUNG

(11) **EP 1 911 397 A2**
(43) Veröffentlichungstag der Anmeldung: **16.04.2008**
(21) Anmeldenummer: 07018662.2
(22) Anmeldetag: 22.09.2007
(51) Int. Cl.: A61B 5/024

(54) **Pulsmesseinrichtung**

(30) Priorität: 09.10.2006 DE 102006047711
(71) Anmelder: Beurer GmbH & Co. KG, 89077 Ulm (DE)
(72) Erfinder: Bühler, Marco, 89077 Ulm (DE); Meternek, Werner, 89233 Neu-Ulm (DE); Merk, Ernst, 89264 Weissenhorn (DE)
(74) Vertreter: Fleck, Hermann-Joseph

(57) **Zusammenfassung**

Die Erfindung bezieht sich auf eine Pulsmesseinrichtung mit einer eine Anzeigeeinheit (13) aufweisenden, von dem Benutzer während sportlicher Aktivität tragbaren mobilen Haupteinheit (10) und einer dieser zur drahtlosen Daten-Übertragungsverbindung zugeordneten Pulserfassungseinrichtung. Unterschiedlichen Benutzungsanforderungen wird dadurch Rechnung getragen, dass die Pulserfassungseinrichtung mehrere Sensoreinheiten (21, 22) unterschiedlichen Aufbaus umfasst, die von dem Benutzer wahlweise anlegbar und mit der Haupteinheit (10) in Daten-Übertragungsverbindung bringbar sind, und dass die Haupteinheit (10) und/oder mindestens eine Sensoreinheit (21, 22) eine Kalibriereinrichtung (11.1) aufweist, mit der eine Kalibrierung mindestens einer der Sensoreinheiten (21, 22) durchführbar ist

## Beschreibung

Die Erfindung bezieht sich auf eine Pulsmesseinrichtung mit einer eine Anzeigeeinheit aufweisenden, von dem Benutzer während sportlicher Aktivität tragbaren mobilen Haupteinheit und einer dieser zur drahtlosen Daten-Übertragungsverbindung zugeordneten Pulserfassungseinrichtung.

Eine Pulsmesseinrichtung dieser Art ist in der DE 90 16 970 U1 angegeben. Diese bekannte Pulsmesseinrichtung zum Messen der Herzschlagfrequenz während einer sportlichen Betätigung weist einen Brustgurt mit einem im Bereich des Herzens angeordneten Pulssensor zum Aufnehmen der Herzpulsfrequenz und Umwandeln der erfassten Signale auf, um diese telemetrisch, und zwar drahtlos an einen Empfangsteil zu übertragen und dort in eine optische und/oder akustische Anzeige für den Benutzer umzuwandeln. Der Empfangsteil ist vorzugsweise ähnlich einer Armbanduhr in einer Einheit am Handgelenk angeordnet und liefert die empfangenen Daten zur Weiterverarbeitung an eine Logikschaltung, insbesondere einen Prozessor, von dem aus die Daten gegebenenfalls über weitere Verarbeitungskomponenten und einen geeigneten Treiber der Anzeigeeinrichtung an dem Handgelenksteil zugeführt werden. Die optische Anzeige weist z.B. eine LCD-Anzeigeeinheit auf, mit der Anzeigesymbole wie Ziffern, Buchstaben und Piktogramme variabel darstellbar sind. Auf diese Weise kann die sich ändernde Herzschlagfrequenz in kurzen Zeitabständen zahlenmäßig dargestellt werden. Auch weist das Handgelenkteil in der Regel eine Speichereinrichtung auf, in der Pulsdaten beispielsweise auch im Zusammenhang mit weiteren Daten, wie Datum und Uhrzeit, speicherbar sind und auch über einen Handeingabeteil eingestellte oder über ein digitales Eingabeteil zugeführte Daten aufgenommen werden können, die bei der Datenverarbeitung mit eingerechnet werden können. Alternativ schlägt die DE 90 16 970 U1 vor, einen Verarbeitungsteil nicht in einem Handgelenkteil anzuordnen, sondern in einem Abschnitt des Brustgurtes selbst, so dass eine telemetrische Datenübertragung zwischen Sendeteil des Brustgurtes und Empfangsteil entfällt, um eine ungestörtere Datenübertragung zu gewährleisten.

Eine Pulsuhr ohne Handgelenkteil ist auch in der EP 0 117 330 A2 beschrieben. Weitere Pulsuhren sind beispielsweise in der DE 693 19 639 T2 und der DE 299 10 633 U1 gezeigt.

Nicht immer entsprechen diese bekannten Pulsmesseinrichtungen den individuellen Komfortwünschen der Benutzer.

Der Erfindung liegt die Aufgabe zugrunde, eine Pulsmesseinrichtung der eingangs genannten Art bereit zu stellen, mit der ein erhöhter Benutzungskomfort erreicht wird.

Diese Aufgabe wird mit den Merkmalen des Anspruches 1 gelöst. Hierbei ist vorgesehen, dass die Pulserfassungseinrichtung mehrere für die Erfassung der Herzpulsfrequenz ausgebildete Sensoreinheiten unterschiedlichen Aufbaus umfasst, die von dem Benutzer wahlweise anlegbar und mit der Haupteinheit in Daten-Übertragungsverbindung bringbar sind, und dass die Haupteinheit und/oder mindestens eine Sensoreinheit eine Kalibriereinrichtung aufweist, mit der eine Kalibrierung mindestens einer der Sensoreinheiten durchführbar ist.

Die verschiedenen Sensoreinheiten ermöglichen es dem Benutzer unter Beibehaltung der einheitlichen mobilen Haupteinheit je nach sportlicher Aktivität oder körperlichen Verfassung wahlweise eine ihm genehme Sensoreinheit zu wählen. Der ihm bekannte Bedienungskomfort bleibt dabei mit der gemeinsamen Haupteinheit erhalten. Auch ist die Aufbewahrung einfach, da im Gegensatz zu mehreren unterschiedlichen Pulsmesseinrichtungen nur wenige Komponenten nach Art eines Bausatzes vorhanden sind. Dabei werden mittels der Kalibrierung zuverlässige Messdaten erzielt, wobei verschiedene Ausgestaltungsmöglichkeiten darin bestehen, dass ein Datenabgleich einmalig bzw. von Zeit zu Zeit erfolgt oder, bei gleichzeitiger längerer Benutzung zweier Sensoreinheiten, öfter oder stetig. Der Kalibriermodus kann dabei automatisch gewählt werden oder manuell. Dabei ist der Ausdruck Kalibriereinrichtung bzw. Kalibrierung allgemein zu verstehen und kann im einfachsten Fall eine Vergleichseinheit zum Vergleich mit einem gespeicherten oder zugeführten Bezugswert für die Weiterverarbeitung oder einfache Ausgabe einer Information auf der Basis des Vergleichsergebnisses beinhalten. Kalibriereinrichtung oder -einheit bzw. Kalibrierung kann auch eine einfache Plausibilisierung des erhaltenen Messwertes bedeuten oder eine Referenzierung anhand eines eindeutigen Bezugswertes.

Zu einem vorteilhaften, einfachen Aufbau tragen dabei die Maßnahmen bei, dass für die Datenübertragung mit allen Sensoreinheiten die Haupteinheit mit einer gemeinsamen Schnittstelle versehen ist.

Die Zuverlässigkeit der Messung wird dabei dadurch unterstützt, dass die Kalibrierung mindestens einer der Sensoreinheiten auf der Grundlage von Daten mindestens einer der anderen Sensoreinheiten erfolgt

Der Aufbau wird weiterhin dadurch begünstigt, dass die Sensoreinheiten einen Datenaufbereitungsteil aufweisen, mit dem erfasste Pulssignale in ein einheitliches Übertragungsprotokoll für die Datenübertragung umwandelbar sind.

Der Datenaufbereitungsteil kann dabei vorteilhaft durch Programmteile und einen Mikrocontroller oder einen anderen integrierten Schaltungsaufbau wie ein ASIC und gegebenenfalls zusätzliche Schaltungskomponenten verwirklicht werden.

Eine einfache Zuordnung und Erkennung der jeweils benutzten Sensoreinheit, die auch in der Anzeige der Haupteinheit kenntlich gemacht werden kann, wird dadurch erreicht, dass das Übertragungsprotokoll eine jeweilige unterschiedliche Kennung für die Sensoreinheiten umfasst.

Verschiedene vorteilhafte Ausgestaltungsmöglichkeiten bestehen darin, dass die Schnittstelle der Haupteinheit mit einem Empfangsteil oder mit einem Sende- und Empfangsteil versehen ist und dass die Sensoreinheiten jeweils mit einer Schnittstelle zum Senden oder einer Schnittstelle zum Senden und Empfangen versehen sind.

Weitere Ausgestaltungsvarianten, mit denen z.B. unterschiedlichen sportlichen Aktivitäten wie Gehen oder Laufen (Wegstreckensensor, Schrittzähler, Rundenzähler oder dgl.), Radfahren (GPS-Einrichtung, Geschwindigkeitsmessung), Schwimmen (Bewegungssensoren) oder Bergsteigen (Höhenmesser, Ortungssensor oder dgl.) Rechnung getragen werden kann, ergeben sich dadurch, dass außer mehreren Sensoreinheiten für die Pulserfassung mindestens eine weitere Sensoreinheit für die Erfassung von Bewegungsparametern und/oder für andere physiologische Messgrößen vorhanden sind, die in gleicher Weise wie die Sensoreinheiten für die Pulserfassung mit der Haupteinheit in Daten-Übertragungsverbindung bringbar und gegebenenfalls erkennbar sind.

Weitere Anschluss- und Datenübertragungsmöglichkeiten werden dadurch erhalten, dass die Haupteinheit außer zur drahtlosen Datenübertragung zusätzlich zur drahtgebundenen Datenübertragung ausgebildet ist und eine dafür ausgebildete zusätzliche Schnittstelle aufweist. Dabei kann es sich um eine standardisierte drahtgebundene Schnittstelle handeln, über die z.B. auch eine Ankopplung der Haupteinheit an einen Auswertecomputer ermöglicht wird.

Verschiedene vorteilhafte Ausgestaltungen bestehen ferner darin, dass die Sensoreinheiten mit einem Verarbeitungsteil zur Datenvorverarbeitung und die Haupteinheit mit einer Verarbeitungseinheit zur Datenverarbeitung für die Auswertung und Anzeige von Benutzerinformationen ausgestaltet sind. Auch hierbei können als Verarbeitungsteil in den Sensoreinheiten bzw. Verarbeitungseinheit in der Haupteinheit Mikrocontroller oder andere digitale Schaltungen gegebenenfalls in Verbindung mit weiteren Komponenten, wie z.B. Speicherkomponenten, eingesetzt werden.

Vorteilhafte Ausgestaltungsmöglichkeiten bestehen darin, dass die Sensoreinheiten einen EKG-Sensor und zumindest einen optischen Sensor, Drucksensor, Ultraschallsensor, Piezosensor, kapazitiven Sensor, induktiven Sensor oder auf elektrischer Impedanzmessung basierenden Sensor aufweisen. Hierbei lässt der EKG-Sensor insbesondere in einem Brustgurt zuverlässige Pulsmessungen zu.

Demgemäß kann bei einer weiteren Ausgestaltung vorgesehen sein, dass die Verarbeitungseinheit der Haupteinheit zur Einbeziehung der Daten des EKG-Sensors zur Kalibrierung und Bewertung der Daten des optischen Sensors, des Drucksensors oder eines der noch genannten Pulssensoren ausgebildet ist.

Weitere vorteilhafte Ausgestaltungen für unterschiedliche Anbringungsstellen am menschlichen Körper bestehen darin, dass der EKG-Sensor in einem Brustgurt integriert ist und dass der optische Sensor als Fingersensor in einem Fingerhalter oder als Ohrsensor in z.B. einem Ohrläppchenhalter gehalten ist.

Für die Zuverlässigkeit der Datenerfassung und Messwertausgabe sind des Weiteren die Maßnahmen von Vorteil, dass der optische Sensor mit einem Temperaturbewertungsteil versehen ist, dass die Temperatursignale in die Vorverarbeitung in dem optischen Sensor und/oder in die Verarbeitung in der Haupteinheit einbezogen sind und dass auf der Basis der Temperatursignale eine Korrektur der Verarbeitung und/oder Vorverarbeitung, eine Gut-/Schlechtbewertung der Pulsmessung und/oder eine Unterdrückung der Pulsmessdaten und/oder eine Ansteuerung einer Stimulationsvorrichtung zur Temperaturerhöhung des Körperbereiches an der Messstelle erfolgt. Entsprechend kann der Temperaturbewertungsteil auch mit einem der genannten anderen Pulssensoren in Zusammenhang gebracht werden.

Die Zuverlässigkeit der Pulsmessungen und der dem Benutzer mitgeteilten Informationen wird dadurch unterstützt, dass auf der Basis der Temperatursignale eine zusätzliche Anzeigefunktion steuerbar ist, mit der auf einen möglichen oder tatsächlichen Fehler hingewiesen wird.

Dabei bestehen verschiedene Ausgestaltungsmöglichkeiten darin, dass zum Erfassen des Temperatursignals ein gesonderter Temperatursensor oder ein Bewertungsteil zum Herleiten der Temperatur aus dem optischen Messsignal vorgesehen ist.

Für die Handhabung und Funktion sind des Weiteren die Maßnahmen von Vorteil, dass der Fingerhalter zum Fixieren eines Sensorelementes des Fingersensors an der Fingerinnenseite nahe der Handfläche ausgebildet ist und eine um den Finger legbare, in ihrem Durchmesser verstellbare Halteschlaufe aufweist.

Dabei wird die Messung dadurch begünstigt, dass der Verarbeitungsteil und die Empfangseinheit des Fingersensors auf dem Fingerrücken angeordnet sind.

Für die Handhabung, Bedienung und die Anzeige von Informationen sind ferner die Maßnahmen vorteilhaft, dass die Haupteinheit in einer Trageeinheit aufgenommen ist, die am Handgelenk anbringbar ist.

Eine zweckmäßige Ausgestaltung, beispielsweise zum Fahrradfahren oder Gehen mit einem Stock, besteht eine vorteilhafte Ausgestaltungsvariante darin, dass die Trageeinheit mit dem Fingerhalter verbunden ist.

Eine weitere vorteilhafte Ausgestaltung insbesondere für die Bedienung und das Ablesen von Anzeigen besteht darin, dass die Haupteinheit herausnehmbar in einer Aufnahme einer an dem Benutzer anlegbaren Einheit fixiert ist und dass eine an das Gehäuse der Haupteinheit angepasste weitere Aufnahme in einer Halterung eines von dem Benutzer für die sportliche Aktivität benutzten Sportgeräts ausgebildet ist, in der die Haupteinheit wahlweise einsetzbar ist.

Verschiedene Ausgestaltungsvarianten für die Informationsausgabe bzw. Bedienung bestehen darin, dass die Haupteinheit mit einer optischen, akustischen und/oder taktilen Anzeigeeinheit sowie einer Bedieneinheit versehen ist.

Die Erfindung wird nachfolgend anhand von Ausführungsbeispielen unter Bezugnahme auf die Zeichnungen näher erläutert. Es zeigen:
- Fig. 1: eine Pulsmesseinrichtung mit einer Haupteinheit und mehreren alternativ oder gemeinsam verwendbaren Sensoreinheiten,
- Fig. 2: eine schematische Block-Darstellung wesentlicher Komponenten der Pulsmesseinrichtung nach Fig. 1,
- Fig. 3: ein weiteres Ausführungsbeispiel der Pulsmesseinrichtung mit einer modifizierten Haupteinheit und mehreren Sensoreinheiten sowie Halteeinheiten,
- Fig. 4: ein Ausführungsbeispiel für eine Sensoreinheit mit Fingerhalter und
- Fig. 5: ein Schaltungsbeispiel für eine Temperaturerfassung in Verbindung mit einer Sensoreinheit.

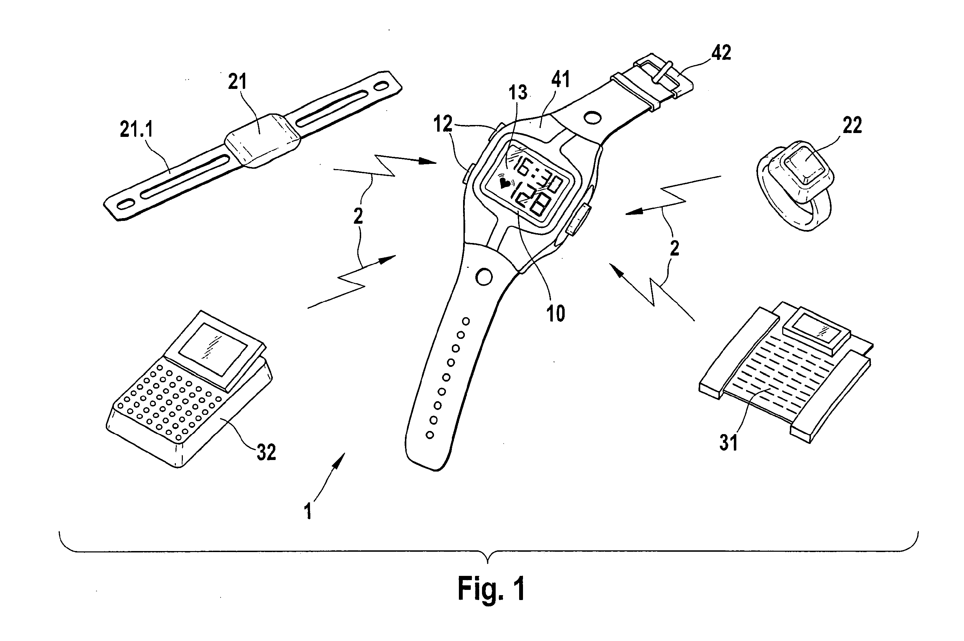

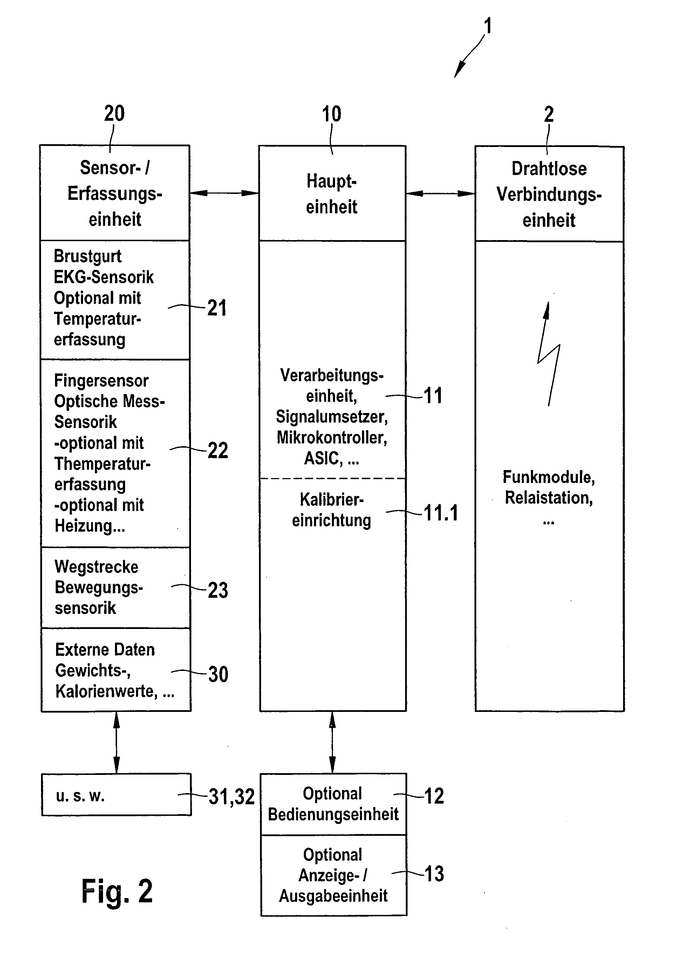

Fig. 1 zeigt eine Pulsmesseinrichtung 1 mit einer am Handgelenk eines Benutzers anlegbaren uhrenähnlichen mobilen Teil mit einer Haupteinheit 10, die in einer Aufnahmeeinheit 41 aufgenommen ist und eine Verarbeitungseinheit 11 (vgl. Fig. 2) sowie eine Bedieneinheit 12 mit mehreren Bedientasten und eine Ausgabeeinheit mit einer Anzeigeeinheit 13 zur Ausgabe von Informationen für den Benutzer aufweist. Die Anzeigeeinheit 13 ist vorliegend als optische Einheit, beispielsweise mit einer LCD-Anzeigeeinrichtung und gegebenenfalls Leuchtdioden ausgebildet und kann zusätzlich auch eine akustische und/oder taktile (z.B. vibratorische) Anzeigeeinheit umfassen. Mit der Haupteinheit 10 stehen mehrere, vorliegend beispielhaft zwei Puls-Sensoreinheiten, nämlich eine EKG-Sensoreinheit 21, die in einem Träger herznah, z.B. an einem Brustgurt 21.1 oder Kleidungsstück integriert ist, und eine optische Sensoreinheit 22 für die Herzpulsmessung, ausgebildet als Fingersensor, sowie zusätzliche Sensoreinheiten 23, wie eine Bewegungssensorik, ein Wegstreckensensor, ein Außentemperatursensor, ein Höhenmesser, ein GPS-Sensor oder dgl., in drahtloser Daten-Übertragungsverbindung über eine Übertragungseinrichtung 2. Darüber hinaus können noch weitere Zusatzeinheiten, insbesondere für stationäre Daten, beispielsweise eine Diätwaage 31 und eine Körperwaage 32 gegebenenfalls ausgebildet als Körperfettwaage, vorgesehen sein, um die mit der Pulsmesseinrichtung erfassten Daten zu ergänzen und eine erweiterte Auswertung unter Einbeziehung der stationären Daten 30 bzw. externen Daten durchzuführen. Die Haupteinheit 10 weist zum Aufnehmen der von den Sensoreinheiten, und gegebenenfalls zusätzlichen Sensoreinheiten sowie evtl. noch vorhandenen Zusatzeinheiten 31, 32 gesendeten Daten eine Empfangs- oder Sende-Empfangsschnittstelle auf, während die Sensoreinheiten 21, 22, gegebenenfalls weiteren Sensoreinheiten 21, 22 und Zusatzeinheiten 31, 32 ihrerseits eine Sende- oder Sende-/Empfangsschnittstelle zur drahtlosen Kommunikation mit der Haupteinheit 10 aufweisen. Zudem kann die Haupteinheit 10 eine Schnittstelle zum Anschluss eines Datenkabels für eine drahtgebundene Datenübertragung aufweisen, an die gegebenenfalls weitere Zusatzgeräte oder Auswertegeräte, wie z.B. ein Auswertecomputer angeschlossen werden können.

In Fig. 2 sind wesentliche Komponenten der Pulsmesseinrichtung 1 in einer Übersicht schematisch in einer Blockdarstellung wiedergegeben. Dabei sind die Sensoreinheiten 21, 22, zusätzlichen Sensoreinheiten 23 und Zusatzeinheiten 31, 32 in einer Ergänzungseinrichtung 20 unter dem Oberbegriff Sensor-/Erfassungseinheit zusammengefasst. Die EKG-Sensoreinheit 21 umfasst die in einem Brustgurt integrierte EKG-Sensorik, optional mit Temperaturerfassung zur genaueren Bewertung der erfassten und übertragenen sowie dann ausgewerteten Signale. Die optische Sensoreinheit 22, ausgebildet als Fingersensor, umfasst eine optische Mess-Sensorik und optional eine Temperaturerfassung und gegebenenfalls Stimulationsvorrichtung, z.B. eine Heizeinrichtung für die Aufwärmung von Hautbezirken im Bereich der Messstelle, wenn diese infolge zu niedriger Temperatur nicht mehr genügend durchblutet sind, um die Signale genau erfassen zu können. Die zusätzlichen Sensoreinheiten 23 umfassen beispielsweise eine Sensorik für die Wegstrecke, eine Bewegungssensorik, mit der Rückschlüsse auf die Aktivität des Benutzers und bei besonderer Ausgestaltung, auch auf richtige Bewegungsabläufe gezogen werden können. Die externen bzw. stationären Daten 30 umfassen z.B. Gewichts- und/oder Kalorienwerte und werden z.B. von der Diätwaage 31 bzw. Körper(fett)waage 32 erhalten.

Die Haupteinheit 10 umfasst eine Verarbeitungseinheit 11 für die von den Sensoreinheiten 21, 22, zusätzlichen Sensoreinheiten 23 und gegebenenfalls Zusatzeinheiten 31, 32 erhaltenen Daten, die schon vor der Datenübertragung in der jeweiligen Sensoreinheit bzw. weiteren Sensoreinheit oder Zusatzeinheit vorverarbeitet sein können. Des Weiteren sind Schaltungskomponenten für die Signalumsetzung und Datenauswertung vorhanden, wobei die Daten dann gegebenenfalls weiterverarbeitet über die Anzeigeeinheit 13 ausgegeben werden. Die Datenverarbeitung in der Haupteinheit 10 sowie die Datenaufbereitung in den Sensoreinheiten 21, 22, weiteren Sensoreinheiten 23 und gegebenenfalls den Zusatzeinheiten 31, 32 erfolgt vorteilhaft mittels jeweiliger Mikrocontroller, die entsprechende Programmteile beinhalten. Auch andere integrierte Schaltungen für die Datenverarbeitung, wie z.B. ASICs oder dgl. sowie elektronische Zusatzkomponenten, wie z.B. Speicherkomponenten, können insbesondere in der Haupteinheit 10 vorhanden sein. Die Verarbeitungseinheit 11 steht über geeignete weitere elektrische Schaltungskomponenten mit der Bedieneinheit 12 bzw. der Ausgabe-/Anzeigeeinheit 13 in Verbindung.

Für die drahtlose Übertragungseinrichtung 2 sind geeignete Funkmodule vorgesehen, wobei zwischen der Haupteinheit 10 und den Sensoreinheiten 21, 22 bzw. weiteren Sensoreinheiten 23 bzw. Zusatzeinheiten 31, 32 für die Datenanpassung Relaisstationen vorgesehen sein können.

Die von den Sensoreinheiten 21, 22, weiteren Sensoreinheiten 23 und gegebenenfalls Zusatzeinheiten 31, 32 aufbereiteten Daten werden für die Übertragung in ein einheitliches Format umgewandelt, so dass sie in gleicher Weise übertragen und von der Haupteinheit 10 eindeutig erkannt und erfasst werden können. Für die einzelnen Sensoreinheiten 21, 22, weiteren Sensoreinheiten 23 und gegebenenfalls Zusatzeinheiten 31, 32 werden dabei jeweilige Kennungen zu ihrer Unterscheidung mit übertragen, die dann auch nach entsprechender Verarbeitung in der Anzeigeeinheit 13 als zusätzliche Information speziell dargestellt werden können.

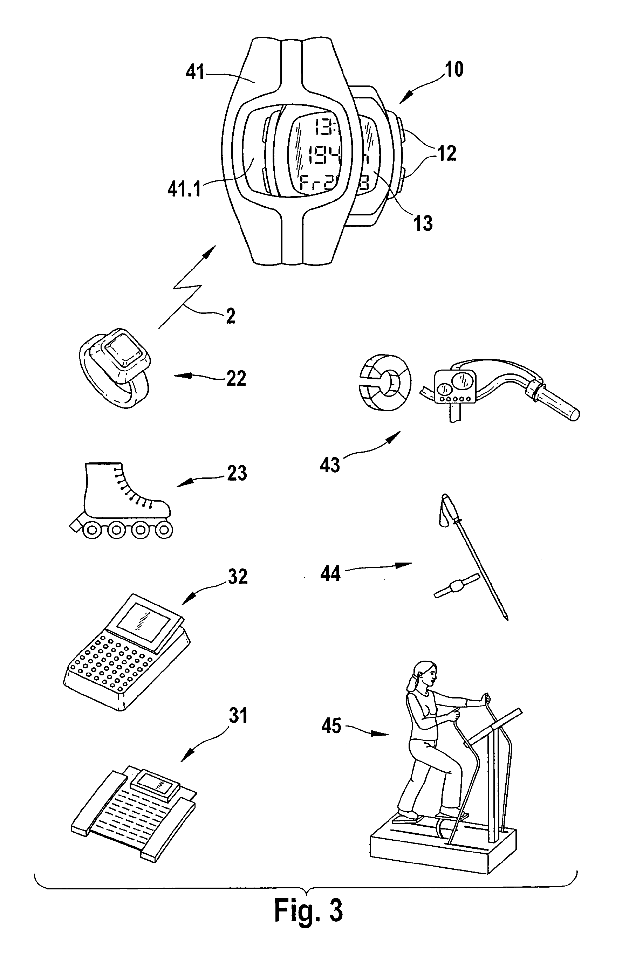

Bei dem in Fig. 3 gezeigten Ausführungsbeispiel ist die Haupteinheit 10 lösbar in einer Aufnahme 41.1 der Aufnahmeeinheit 41 fixiert und kann herausgenommen werden, um sie z.B. in einer mit einer entsprechenden Aufnahme versehenen Fahrradhalterung 43, Stockhalterung 44 oder einer anderen Trainingsgerätehalterung 45 je nach Art der sportlichen Betätigung einzusetzen. Auf diese Weise wird ein einfaches Ablesen einer optischen Anzeige der Anzeigeeinheit 13 bzw. ein einfaches Bedienen begünstigt. Zudem ist gezeigt, dass eine weitere Sensoreinheit 23 z.B. in einem Sportschuh integriert sein kann, um die Bewegung zu registrieren oder Schritte zu zählen.

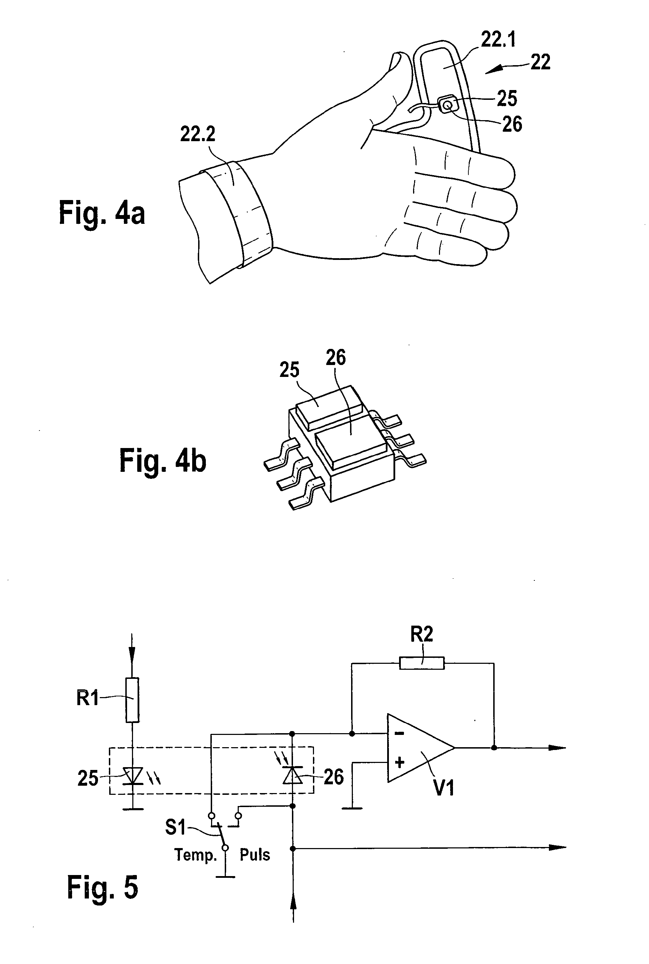

Wie aus Fig. 4 ersichtlich, ist die optische Sensoreinheit 22, die als Fingersensor ausgebildet ist, in einem um den Finger des Benutzers legbaren, an den Umfang anpassbaren schlaufenartigen Fingerhalter 22.1 integriert. Der Fingerhalter weist zum Festlegen vorteilhaft einen Klettverschluss oder z.B. Druckknopfverschluss oder dgl. auf. Der Sensor wird mit dem Fingerhalter 22.1 vorzugsweise auf der Innenseite des Fingers nahe der Handfläche fixiert, während ein Verarbeitungsteil für die Aufbereitung der erfassten optischen Signale und der Sendeteil beispielsweise im Bereich des Fingerrückens gehalten werden. Zudem kann der bandförmige Fingerhalter 22.1 mit einem Armhalter 22.2 verbunden sein, der auch die Haupteinheit 10 im Handgelenkbereich oder auf dem Handrücken tragen kann. Mit diesem Aufbau wird eine sichere Fixierung, insbesondere des Sensorelements erreicht, wobei die Sensoreinheit 22 auf der Innenseite des Fingers möglichst wenig stört. Zudem ist in Fig. 4 auch beispielhaft eine optische Sendeeinheit 25 und eine optische Empfangseinheit 26 der optischen Sensoreinheit 22 gezeigt, die in einem Bauteil zusammengefasst sind.

In Fig. 5 ist ein Ausführungsbeispiel für eine Temperaturerfassung bei der Pulserfassung mit der optischen Sensoreinheit 22 dargestellt. In einem Sendezweig mit der optischen Sendeeinheit 25 und einem dieser vorgeschalteten ersten Widerstand R1 wird eine optische Strahlung abgegeben, die Blutgefäße in dem betreffenden Bereich des Fingers durchleuchtet oder an diesen reflektiert wird und anschließend von der optischen Empfangseinheit 26 empfangen wird. Daraufhin wird das Signal mittels eines Verstärkers V1, beispielsweise eines Operationsverstärkers, der über einen zweiten Widerstand R2 rückgekoppelt ist, verstärkt. Zudem kann durch automatisch gesteuertes Umschalten einer Schalteinheit S1 ein Temperatursignal erfasst werden. Die Temperaturerfassung kann z.B. auf der Basis einer temperaturabhängigen Änderung der spektralen Verteilung, der Strahlungsleistung oder mittels separater Temperatursensoren erfolgen.

Die Haupteinheit kann auch an einer anderen geeigneten Körperstelle, beispielsweise am Oberarm, mittels einer entsprechend ausgebildeten Trageeinheit getragen werden. Die Sensoreinheiten können auch zur Pulserfassung auf der Basis anderer physikalischer Größen beruhen, wie z.B. Druck, der z.B. mittels Dehnungsstreifen erfasst werden kann, elektrischer Impedanz, Ultraschall, und es können zur Erfassung z.B. Piezotechnik (Piezofolien oder dgl.) oder kapazitive oder induktive Sensoren eingesetzt werden. Dabei kann die Signalerfassung außer an der Fingerinnenseite z.B. am Ohrläppchen, an der Handinnenfläche, an der Fingernagelwurzel oder in der Nähe des Handgelenks oder am Fuß oder einer anderen geeigneten Körperstelle erfolgen.

Generell wird über die Erfassung des EKG-Signals, insbesondere mittels eines Brustgurts, eine sehr zuverlässige Messung erhalten, die zur Kalibrierung der anderen Sensoreinheiten herangezogen werden kann, so dass mittels der Verarbeitungseinrichtung der Haupteinheit 10 eine entsprechende Bewertung der von den anderen Sensoreinheiten erhaltenen Puls-Daten durchgeführt werden kann. Hierzu weist die Verarbeitungseinheit 11 eine mittels eines Programmteils oder Hardware realisierte Kalibriereinrichtung 11.1 auf, die zu einem ständigen oder von Zeit zu Zeit durchgeführten Datenabgleich ausgebildet sind, falls beide Sensoreinheiten gleichzeitig benutzt werden, oder zu einem gelegentlichen Datenabgleich. Dabei kann die Kalibrierung 11.1 zur manuellen oder automatischen Auslösung des Kalibriervorganges ausgebildet sein.

Auch kann die Verarbeitungseinheit 11 der Haupteinheit 10 so ausgebildet sein, dass sie Unregelmäßigkeiten bzw. Abweichungen in den mit den Sensoreinheiten erfassten Signalen erkennt und ein Fehlersignal bei Abweichung von einem Bezugssignal bzw. Sollwert ausgibt oder eine Korrektur vornimmt. Wird mittels der Verarbeitungseinheit 11 oder bereits in einem betreffenden Verarbeitungsteil der Sensoreinheit erkannt, dass eine Verfälschung oder ein schwaches Pulssignal auf eine zu niedrige Temperatur und damit schlechte Durchblutung der Erfassungsstelle zurückzuführen ist, so kann die betreffende Stelle der Haut gereizt bzw. mittels eines Wärmeelementes angewärmt werden.

Bei einer erweiterten Ausgestaltung des Kalibrierungseinrichtung 11.1 kann vorgesehen sein, dass die Kalibrierung nicht notwendigerweise auf der Basis des EKG-Signals erfolgt, sondern auf der Basis zweier beliebigen Puls-Sondeneinheiten untereinander.

Auch kann eine Kalibriereinrichtung oder eine zusätzliche Kalibriereinrichtung in einer oder mehreren Sensoreinheiten 21, 22 angeordnet sein. Der Ausdruck Kalibriereinrichtung bzw. Kalibrierung ist hierbei allgemein zu verstehen und kann bei einfacher Ausführung eine Vergleichseinheit bzw. Vergleichsvorgang mit einem oder mehreren gespeicherten oder zugeführten Bezugswerten bedeuten oder bei aufwändiger Gestaltung eine Verarbeitung zum Durchführen eines algorithmischen Abgleichs. Auch ist eine Ausgestaltung der Kalibrierung als Referenzierung denkbar.

Durch Vergleich des EKG-Signals mit den Signalen von an den Extremitäten angeordneten Sensoren, können z.B. auch Arterienwiderstände im Körper oder unbekannte Arterienerkrankungen und dadurch verursachte Verfälschungen erkannt werden. Gegenüber einer EKG-Messung z.B. mit Brustgurt hat eine Pulsmessung an einer Extremität den Vorteil, dass die Sensoreinheit leichter anzulegen ist und eine Schweißbildung im Brustbereich, wie sie durch einen Brustgurt verursacht werden kann, vermieden wird. Bei einem Fingersensor ist der optimale Messort der rechte oder linke Mittelfinger an der Handinnenfläche mit den dort vorhandenen Makro- bzw. Mikroblutgefäßen und wegen eines möglichst geringen Kälteeinflusses.

Für die optische Messung eignet sich z.B. ein Reflexionsmessverfahren mit einer optischen IR-Wellenlänge, wobei die Messung mit Licht zusätzlicher Wellenlängen verbessert werden kann. Durch Erwärmung der betreffenden Körperstellen mittels Wärmeelementen oder durch Wärmeisolation mit einem Handschuh können relativ konstante Messbedingungen erreicht werden. Für eine Heizung kommen in Betracht eine IR-Strahlungsquelle, insbesondere IR-LED, Wärmewiderstände, Verlustwärme anderer elektrischer Bauteile, beheizte Textilien, wie z.B. ein Fingerhandschuh, oder auch ein beheiztes Miniflüssigbehältnis mit Gel und dgl. Als Temperaturmesselemente kommen Dioden, NTC-/PTC-Widerstände, veränderbare Widerstände, Widerstandsdrähte, Thermoelemente oder Bestimmung der Farbe (Farbtemperatur) an der Hautoberfläche durch optische Messverfahren in Betracht.

Der Energieverbrauch der optischen Messeinheit für die Pulsmessung lässt sich durch eine gepulste Ansteuerung der Lichtquelle oder eines mit dieser zusammenarbeitenden Mikrocontrollers erreichen, wobei eine Steuerung oder Regelung der Strahlung z.B. in Abhängigkeit von dem Messort, der Umgebungshelligkeit oder der Temperatur vorgenommen werden kann. Mit diesen Maßnahmen kann die Betriebslebensdauer der Messeinrichtung und ihrer Energiequelle erheblich erhöht werden.

Prinzipiell muss die IR-Strahlungsleistung bei kälteren Temperaturen oder hellerem Tageslicht erhöht werden.

Weitere Maßnahmen betreffen die Bestimmung der zeitabhängigen Blutflussmenge im oder außerhalb des Sensorbereichs, um Rückschlüsse auf das zu erwartende Messsignal zu ziehen, beispielsweise ob eine Hypothermie, schlechte Durchblutung oder anderer Einfluss vorliegt. Diese Bestimmung kann auch alternativ zur Temperaturerfassung herangezogen werden. Weitere Aussagen lassen sich durch Ermittlung mehrerer biologischer oder physikalischer Werte, wie z.B. Wasser-, Fett-, Sauerstoffanteil im Gewebe oder Blut z.B. durch elektrische Impedanzmessung und/oder Sauerstoffatmungssensorik gewinnen. Auch kann z.B. kontrolliert werden, ob Puls- und Atmungsfrequenz im Gleichklang sind, um auf diese Weise eine Biofeedback-Aussage zu erhalten. Auch kann die Pulserfassung zwei- oder mehrfach unter optischer Durchlichtmessung oder Reflexionsmessung und Körperimpedanzmessung bzw. EKG-Signalmessung erfolgen. Auf diese Weise kann auch eine schnellere Pulswertbestimmung und Erkennung von Arrythmien bzw. Herzrhythmusstörungen oder Erkennung von Pulswertunterschieden z.B. durch verengte oder verhärtete Arterien erfolgen. Diese zusätzlichen Maßnahmen können mittels entsprechender programmierter oder programmierbarer Algorithmen in der Verarbeitungseinheit 11 durchgeführt werden.

Ferner ist vorteilhaft eine generelle Messwertbereichskalibrierung von Zeit zu Zeit automatisch oder durch Wahl des Benutzers insbesondere unter Einbeziehung der Temperatur vorgesehen, wodurch genauere Messwerte und Informationen für den Benutzer erhalten werden.

Für die Befestigung der Sensoreinheiten 21, 22 ist die Halterung für einen geringen, aber nicht zu schwachen Anpressdruck ohne Blutstau oder Erzeugung eines Taubheitsgefühls beim Tragen ausgelegt, wobei insbesondere beim Fingersensor eine kleine Flächenkontaktierung vorgesehen ist, um großen Bewegungsfreiraum aller Finger zuzulassen. Dabei ist der Aufbau flach gestaltet und eine einfache Anlegetechnik und sichere Fixierung vorgesehen. Ferner sind Materialien ohne allergisierende Wirkungen gewählt.

Zur Befestigung des Fingersensors bestehen verschiedene Ausgestaltungsmöglichkeiten in einer ringförmig geschlossenen Ausführung, einer Teilöffnung mit Klemmfunktion, einem Gummiband, einem Klettverschluss, einem Band mit Magnetverschluss, einer Luftbeutelfixierung durch Aufpumpen bzw. in einer Zug-/Druckfeder zur Teilbereichsanpressung. Auch eine Teilbereichsanpressung mittels Einstellschraube, Rastscharnier mit Schnellöffnung, Kettenglieder mit starren oder elastischen Bändern, Klettverschlüssen, Wickelverschlüssen oder dgl. sind anwendbar.

Außer der Anzeige auf einer Anzeigefläche der Anzeigeeinheit 13 kommen z.B. neben einer akustischen Kennzeichnung der Pulsfrequenz durch intermittierende Töne gegebenenfalls angepasster Frequenz optische Projektionseinrichtungen, wie z.B. ein Laserstrahl bzw. LED-Strahlenbündel in Betracht, die auf umgebende Gegenstände treffen, womit gleichzeitig auch eine erhöhte Sicherheit im Verkehr erreicht werden kann. Auch können Warnanzeigen für anormale Zustände optisch, akustisch und/oder taktil codiert angezeigt werden. Für die akustische Anzeige kommen insbesondere auch geeignete Sprachausgaben in Betracht, die über die Haupteinheit 10 gesteuert und ausgegeben werden.

Der Informationsinhalt der Anzeigen kann sich neben der Angabe der Pulswerte auf verschiedene andere Informationen, wie Datum, Uhrzeit, Anzeige der Wegstrecke Rundenanzeige oder zusätzlicher Anzeigen beispielsweise in Verbindung mit einem Fahrradcomputer beziehen. Auch können geeignete Informationen ausgegeben werden, die ein nicht korrektes Anlegen der Sensoreinheiten 21, 22 betreffen.

## Patentansprüche

1. Pulsmesseinrichtung mit einer eine Anzeigeeinheit (13) aufweisenden, von dem Benutzer während sportlicher Aktivität tragbaren mobilen Haupteinheit (10) und einer dieser zur drahtlosen Daten-Übertragungsverbindung zugeordneten Pulserfassungseinrichtung,
**dadurch gekennzeichnet,**
**dass** die Pulserfassungseinrichtung mehrere zur Herzpulsfrequenzerfassung ausgebildete Sensoreinheiten (21, 22) unterschiedlichen Aufbaus umfasst, die von dem Benutzer wahlweise anlegbar und mit der Haupteinheit (10) in Daten-Übertragungsverbindung bringbar sind, und
**dass** die Haupteinheit (10) und/oder mindestens eine Sensoreinheit (21, 22) eine Kalibriereinrichtung (11.1) aufweist, mit der eine Kalibrierung mindestens einer der Sensoreinheiten (21, 22) durchführbar ist.

2. Pulsmesseinrichtung nach Anspruch 1,
**dadurch gekennzeichnet,**
**dass** die Kalibrierung mindestens einer der Sensoreinheiten (21, 22) auf der Grundlage von Daten mindestens einer der anderen Sensoreinheiten (22, 21) erfolgt.

3. Pulsmesseinrichtung nach Anspruch 1 oder 2,
**dadurch gekennzeichnet,**
**dass** für die Datenübertragung mit allen Sensoreinheiten (21, 22) die Haupteinheit (10) mit einer gemeinsamen Schnittstelle versehen ist.

4. Pulsmesseinrichtung nach einem der vorhergehenden Ansprüche,
**dadurch gekennzeichnet,**
**dass** die Sensoreinheiten (21, 22) einen Datenaufbereitungsteil aufweisen, mit dem erfasste Pulssignale in ein einheitliches Übertragungsprotokoll für die Datenübertragung umwandelbar sind.

5. Pulsmesseinrichtung nach Anspruch 4,
**dadurch gekennzeichnet,**
**dass** das Übertragungsprotokoll eine jeweilige unterschiedliche Kennung für die Sensoreinheiten (21, 22) umfasst.

6. Pulsmesseinrichtung nach einem der Ansprüche 3 bis 5,
**dadurch gekennzeichnet,**
**dass** die Schnittstelle der Haupteinheit (10) mit einem Empfangsteil oder mit einem Sende- und Empfangsteil versehen ist und
**dass** die Sensoreinheiten (21, 22) jeweils mit einer Schnittstelle zum Senden oder einer Schnittstelle zum Senden und Empfangen versehen sind.

7. Pulsmesseinrichtung nach einem der vorhergehenden Ansprüche,
**dadurch gekennzeichnet,**
**dass** außer mehreren Sensoreinheiten (21, 22) für die Pulserfassung mindestens eine weitere Sensoreinheit für die Erfassung von Bewegungsparametern und/oder für andere physiologische Messgrößen vorhanden sind, die in gleicher Weise wie die Sensoreinheiten für die Pulserfassung mit der Haupteinheit (10) in Daten-Übertragungsverbindung bringbar sind.

8. Pulsmesseinrichtung nach einem der vorhergehenden Ansprüche,
**dadurch gekennzeichnet,**
**dass** die Haupteinheit (10) außer zur drahtlosen Datenübertragung zusätzlich zur drahtgebundenen Datenübertragung ausgebildet ist und eine dafür ausgebildete zusätzliche Schnittstelle aufweist.

9. Pulsmesseinrichtung nach einem der vorhergehenden Ansprüche,
**dadurch gekennzeichnet,**
**dass** die Sensoreinheit (21, 22) mit einem Verarbeitungsteil zur Datenvorverarbeitung und die Haupteinheit (10) mit einer Verarbeitungseinheit (11) zur Datenverarbeitung für die Auswertung und Anzeige von Benutzerinformationen ausgestaltet sind.

10. Pulsmesseinrichtung nach einem der vorhergehenden Ansprüche,
**dadurch gekennzeichnet,**
**dass** die Sensoreinheiten einen EKG-Sensor (21) und zumindest einen optischen Sensor, Drucksensor, Ultraschallsensor, Piezosensor, kapazitiven Sensor, induktiven Sensor oder auf elektrischer Impedanzmessung basierenden Sensor aufweisen.

11. Pulsmesseinrichtung nach Anspruch 10,
**dadurch gekennzeichnet,**
**dass** die Verarbeitungseinheit (11) zur Einbeziehung der Daten des EKG-Sensors (21) zur Kalibrierung und Bewertung der Daten des optischen Sensors (22) und/oder des Drucksensors ausgebildet ist.

12. Pulsmesseinrichtung nach Anspruch 10 oder 11,
**dadurch gekennzeichnet,**
**dass** der EKG-Sensor (21) in einem Brustgurt (21.1) integriert ist und
**dass** der optische Sensor als Fingersensor (22) in einem Fingerhalter (22.1) oder einem Ohrläppchenhalter gehalten ist.

13. Pulsmesseinrichtung nach Anspruch 12,
**dadurch gekennzeichnet,**
**dass** der optische Sensor (22) mit einem Temperaturbewertungsteil in Verbindung gebracht ist,
**dass** die Temperatursignale in die Vorverarbeitung in dem optischen Sensor (22) und/oder in die Verarbeitung in der Haupteinheit (10) einbezogen sind und
**dass** auf der Basis der Temperatursignale eine Korrektur der Verarbeitung und/oder Vorverarbeitung, eine Gut-/Schlechtbewertung der Pulsmessung und/oder eine Unterdrückung fehlerhafter Pulsmessdaten und/oder eine Ansteuerung einer Stimulationsvorrichtung zur Temperaturerhöhung des Körperbereiches an der Messstelle erfolgt.

14. Pulsmesseinrichtung nach Anspruch 13,
**dadurch gekennzeichnet,**
**dass** auf der Basis der Temperatursignale eine zusätzliche Anzeigefunktion steuerbar ist, mit der auf einen möglichen oder tatsächlichen Fehler hingewiesen wird.

15. Pulsmesseinrichtung nach Anspruch 13 oder 14,
**dadurch gekennzeichnet,**
**dass** zum Erfassen des Temperatursignals ein gesonderter Temperatursensor oder ein Bewertungsteil zum Herleiten der Temperatur aus dem optischen Messsignal vorgesehen ist.

16. Pulsmesseinrichtung nach einem der Ansprüche 12 bis 15,
**dadurch gekennzeichnet,**
**dass** der Fingerhalter (22.1) zum Fixieren eines Sensorelementes des Fingersensors (22) an der Fingerinnenseite nahe der Handfläche ausgebildet ist und eine um den Finger legbare, in ihrem Durchmesser verstellbare Halteschlaufe aufweist.

17. Pulsmesseinrichtung nach Anspruch 16,
**dadurch gekennzeichnet,**
**dass** der Verarbeitungsteil und die Empfangseinheit des Fingersensors (22) auf dem Fingerrücken angeordnet sind.

18. Pulsmesseinrichtung nach einem der vorhergehenden Ansprüche,
**dadurch gekennzeichnet,**
**dass** die Haupteinheit (10) in einer Trageeinheit (42) aufgenommen ist, die am Handgelenk anbringbar ist.

19. Pulsmesseinrichtung nach Anspruch 18,
**dadurch gekennzeichnet,**
**dass** die Trageeinheit (42) mit dem Fingerhalter (22.1) verbunden ist.

20. Pulsmesseinrichtung nach einem der vorhergehenden Ansprüche,
**dadurch gekennzeichnet,**
**dass** die Haupteinheit (10) herausnehmbar in einer Aufnahme (41.1) einer an dem Benutzer anlegbaren Einheit fixiert ist und
**dass** eine an die Haupteinheit (10) angepasste weitere Aufnahme in einer Halterung (43, 44) eines von dem Benutzer für die sportliche Aktivität benutzten Sportgeräts ausgebildet ist, in der die Haupteinheit (10) wahlweise einsetzbar ist.

21. Pulsmesseinrichtung nach einem der vorhergehenden Ansprüche,
**dadurch gekennzeichnet,**
**dass** die Haupteinheit (10) mit einer optischen, akustischen und/oder taktilen Anzeigeeinheit sowie einer Bedieneinheit (12) versehen ist.
